# EUROPÄISCHE PATENTSCHRIFT

(11) **EP 1 636 162 B1**
(45) Veröffentlichungstag und Bekanntmachung des Hinweises auf die Patenterteilung: **06.09.2006**
(21) Anmeldenummer: 04733248.1
(22) Anmeldetag: 15.05.2004
(51) Int. Cl.: C07C 51/31, C07C 51/265, C07D 307/89, C07C 51/21

(54) **VERFAHREN ZUR HERSTELLUNG VON PHTHALSÄUREANHYDRID**
METHOD FOR PRODUCING PHTHALIC ANHYDRIDE
PROCEDE DE PRODUCTION D'ANHYDRIDE D'ACIDE PHTALIQUE

(30) Priorität: 23.05.2003 DE 10323817
(43) Veröffentlichungstag der Anmeldung: 22.03.2006
(73) Patentinhaber: BASF Aktiengesellschaft, 67056 Ludwigshafen (DE)
(72) Erfinder: NETO, Samuel, 68161 Mannheim (DE); ZÜHLKE, Jürgen, 67346 Speyer (DE); STORCK, Sebastian, 68167 Mannheim (DE); ROSOWSKI, Frank, 68165 Mannheim (DE)
(86) Internationale Anmeldenummer: PCT/EP2004/005246
(87) Internationale Veröffentlichungsnummer: WO 2004/103944

(56) Entgegenhaltungen:
- EP-A- 0 985 648
- DE-A- 19 823 262

## Beschreibung

Die Erfindung betrifft ein Verfahren zur Herstellung von Phthalsäureanhydrid durch katalytische Gasphasenoxidation von Xylol und/oder Naphthalin mit einem molekularen Sauerstoff enthaltenden Gas in einem Festbett und mittels mindestens drei in Schichten übereinander angeordneter Katalysatoren, deren Aktivität von Schicht zu Schicht von der Gaseintrittsseite zur Gasaustrittsseite ansteigt, auf deren Kern aus Trägermaterial eine Schicht aus katalytisch aktiven Metalloxiden aufgebracht ist.

Phthalsäureanhydrid wird technisch durch katalytische Gasphasenoxidation von o-Xylol oder Naphthalin in Rohrbündelreaktoren hergestellt. Ausgangsmaterial ist ein Gemisch aus einem molekularen Sauerstoff enthaltenden Gas, beispielsweise Luft, und dem zu oxidierenden o-Xylol und/oder Naphthalin. Das Gemisch wird durch eine Vielzahl in einem Reaktor angeordneter Rohre (Rohrbündelreaktor) geleitet, in denen sich eine Schüttung mindestens eines Katalysators befindet. In den letzten Jahren ist man dazu übergegangen, unterschiedlich aktive Katalysatoren schichtweise in der Katalysatorschüttung anzuordnen, wobei sich in der Regel der weniger aktive Katalysator zum Gaseintritt hin in der obersten, ersten Katalysatorschicht und der aktivere Katalysator zum Gasaustritt hin in der untersten, letzten Katalysatorschicht befindet. Dies wurde anfangs in zwei übereinander liegenden Schichten, einer Ober- und einer Unterschicht realisiert (u. a. DE-A 40 13 051, DE-A 197 07 943, DE-A 25 46 268, US 4,469,878, EP-A 539 878, EP-A 286 448, EP-A 906 783). Seit den letzten Jahren wird vorwiegend ein Katalysatorsystem bestehend aus drei übereinanderliegenden Schichten, einer Ober-, Mittel- und Unterschicht, verwendet. Mit dieser Maßnahme kann das Katalysatorsystem im Reaktor in seiner Aktivität dem Reaktionsverlauf entsprechend angepasst werden. In der unteren Schicht zum Gasaustritt hin gelegen wird dabei vornehmlich restliches o-Xylol oder Naphthalin und Zwischenprodukte wie beispielsweise o-Tolylaldehyd und Phthalid zu Phthalsäureanhydrid umgesetzt. Weiterhin werden aber auch Nebenprodukte wie beispielsweise Chinone weiteroxidiert.

Die Möglichkeiten der Aktivitätsstrukturierung sind dabei sehr vielfältig. Eine besondere Beachtung findet in der folgenden Zusammenstellung des Stands der Technik die letzte Katalysatorschicht, insbesondere deren Phosphor- und Vanadiumgehalte und das Verhältnis zueinander (berechnet als V₂O₅ zu P). Trotz vielfacher Variation dieser Wertepaare im Stand der Technik gibt es noch Optimierungsbedarf in bezug auf die Aktivität und Selektivität der letzten Katalysatorschicht.

Die DE-A 198 23 262 beschreibt ein Verfahren zur Herstellung von Phthalsäureanhydrid mit drei in Schichten übereinander angeordnet Schalenkatalysatoren, wobei die Katalysatoraktivität von Schicht zu Schicht von der Gaseintrittsseite zur Gasaustrittsseite ansteigt und durch die Menge der aufgebrachten Aktivmasse und der Alkalidotierung gesteuert wird. In den Beispielen wird Phosphor in der zweiten und in der dritten Schicht verwendet. Vanadiumpentoxid wird mit weniger als 10 Gew.-% in der letzten Schicht verwendet, das Verhältnis von V₂O₅ zu P liegt bei 37.

EP-A 985 648 beschreibt die Herstellung von Phthalsäureanhydrid durch katalytische Gasphasenoxidation von o-Xylol und/oder Naphthalin mit einem Katalysatorsystem, welches so strukturiert ist, dass die "Porosität" des Katalysators und damit die Aktivität vom Reaktoreingang zum Reaktorausgang hin quasi kontinuierlich ansteigt. Die Porosität wird durch das freie Volumen zwischen den beschichteten Formkörpern der Schüttung im Reaktionsrohr definiert. In den Beispielen wird die Aktivitätsstrukturierung durch Hintereinanderschaltung von mindestens drei verschiedenen Katalysatoren realisiert, wobei die ersten zwei Schichten phosphorfrei sind und die Unterschicht 0.65 bis 0.87 Gew.-% Phosphor und 15 bis 20 Gew.-% Vanadiumpentoxid mit einem V₂O₅ / P Verhältnis von 17 bis 31 aufweist. Die BET-Oberfläche der katalytisch aktiven Komponenten beträgt 70 bis 160 m²/g.

Die WO 03/70680 beschreibt ein mehrzoniges Katalysatorsystem. Die Aktivitätsstrukturierung erfolgt über die Aktivmassenmenge auf dem Träger, der Aktivmasse zugesetzten Menge an Dotierungen in Form von Alkalimetallverbindungen und über die Temperaturführung. Es wird beschrieben, dass die letzten beiden Schichten Phosphor enthalten, dass in der letzten Schicht 6 bis 9 Gew.-% Vanadiumpentoxid enthalten sind und dass das Verhältnis von V₂O₅ zu P zwischen 12 und 90 liegt.

In der EP-A 1063222 wird der Einsatz von drei oder mehrlagigen Katalysatoren beschrieben. Die Aktivität der einzelnen Zonen wird durch die Phosphormenge der Aktivmasse, die Menge der Aktivmasse auf dem Trägerring, die Menge der Alkalidotierung der Aktivmasse und der Füllhöhe der einzelnen Katalysatorlagen im Reaktionsrohr verändert. In den Beispielen werden Katalysatorsysteme beschrieben, die in allen Schichten Phosphor enthalten, die letzte Schicht weist 0,1 bis 0,4 Gew.-% Phosphor, ca. 5 Gew.-% Vanadium (umgerechnet zu V₂O₅) und ein Verhältnis von V₂O₅ zu P von 14 bis 40 auf.

Die Aktivitätssteigerung kann demnach durch folgende Maßnahmen oder Kombinationen hieraus erfolgen:
(1) durch stetigen Anstieg des Phosphorgehalts,
(2) durch stetigen Anstieg des Aktivmassengehalts,
(3) durch stetige Abnahme des Alkaligehalts,
(4) durch stetige Abnahme des Leerraumes zwischen den einzelnen Katalysatoren
(5) durch stetige Abnahme des Gehalts an Inertstoffen oder
(6) durch stetige Zunahme der Temperatur
von der Oberschicht (Gaseintritt) zur Unterschicht (Gasaustritt).

Durch Alterungsprozesse verlieren alle Katalysatoren mit zunehmender Lebenszeit an Aktivität. Dies wirkt sich vorwiegend in der Hauptreaktionszone (Oberschicht) aus, da dort die höchste Temperaturbelastung stattfindet. Die Hauptreaktionszone wandert dabei im Laufe der Katalysatorlebenszeit immer tiefer in das Katalysatorbett. Hieraus resultiert, dass Zwischen- und Nebenprodukte nicht mehr vollständig umgesetzt werden können, da sich die Hauptreaktionszone nun auch in Katalysatorzonen befindet, die weniger selektiv und verstärkt aktiv sind. Die Produktqualität des erzeugten Phthalsäureanhydrids verschlechtert sich somit zunehmend. Dem Rückgang der Umsetzung und damit der Verschlechterung der Produktqualität kann zwar durch Erhöhung der Reaktionstemperatur, beispielsweise mittels Erhöhung der Salzbadtemperatur entgegengewirkt werden. Diese Temperaturerhöhung ist allerdings mit einem Rückgang der Ausbeute des Phthalsäureanhydrids verbunden.

Das Vorhandensein von Zwischen- und Nebenprodukten ist weiterhin umso größer, je höher die Beladung der Luft mit dem zu oxidierenden Kohlenwasserstoff ist, da eine hohe Beladung das Wandern der Hauptreaktionszone tiefer in das Katalysatorbett verstärkt. Für eine wirtschaftliche Herstellung sind aber hohe Beladungen von 60 bis 120 g/Nm³ erwünscht.

Die bei der Alterung, insbesondere im Zusammenhang mit einer hohen Beladung, zunehmenden Nebenprodukte umfassen nicht nur Phthalid (PHD), sondern auch insbesondere Anthrachinondicarbonsäure (ADCA) und Benzoylphthalsäureanhydrid (Benzoyl-PSA), sowie nicht umgesetztes o-Xylol. Im Bezug auf die Verringerung von PHD konnten Verbesserungen erzielt werden (beispielsweise DE-A-198 23 262). Hinsichtlich der allgemeinen Nebenproduktbildung besteht allerdings weiterhin Optimierungsbedarf, insbesondere in Hinblick auf ADCA, da Spuren dieser Verbindungen zu einer Gelbfärbung des Phthalsäureanhydrids führen. Eine Reduzierung dieser Nebenprodukte würde darüber hinaus die Aufarbeitung des Roh-Phthalsäureanhydrids erleichtern.

Der vorliegenden Erfindung lag daher die Aufgabe zugrunde, ein Verfahren zur Herstellung von Phthalsäureanhydrid zur Verfügung zu stellen, das trotz hoher Beladung Phthalsäureanhydrid mit verbesserter Produktqualität bei gleichbleibender oder verbesserter Ausbeute liefert. Neben der Verringerung des gesamten Nebenproduktgehaltes soll insbesondere das Nebenprodukt ADCA bei gleichbleibender oder verbesserter Ausbeute reduziert werden.

Überraschenderweise wurde nun gefunden, dass diese Aufgabe gelöst werden konnte mittels eines Verfahrens zur Herstellung von Phthalsäureanhydrid durch katalytische Gasphasenoxidation von Xylol und/oder Naphthalin mit einem molekularen Sauerstoff enthaltenden Gas in einem Festbett und mittels mindestens drei in Schichten übereinander angeordneter Katalysatoren, deren Aktivität von Schicht zu Schicht von der Gaseintrittsseite zur Gasaustrittsseite ansteigt, auf deren Kern aus Trägermaterial eine Schicht aus katalytisch aktiven Metalloxiden aufgebracht ist, das dadurch gekennzeichnet ist, dass nur die letzte Katalysatorschicht Phosphor aufweist, dass in der letzten Schicht mindestens 10 Gew.-% Vanadium (berechnet als V₂O₅) bezogen auf die Aktivmasse des Katalysators vorliegen und dass das Verhältnis Vanadium (berechnet als V₂O₅) zu Phosphor einen Wert von größer als 35 aufweist.

Die oberen Katalysatorschichten sind phosphorfrei. Bevorzugt weist die letzte, unterste Katalysatorschicht einen Phosphorgehalt von weniger als 1 Gew.-% (berechnet als P) bezogen auf die Aktivmasse des Katalysators auf, bevorzugt weniger als 0,5 Gew.-%, insbesondere etwa 0,05 bis 0,4 Gew.-%.

Bevorzugt beträgt die Schüttungslänge der letzten Katalysatorschicht weniger als 40 % der Gesamtkatalysatorschüttungslänge aller Schichten; insbesondere beträgt die Schüttungslänge der letzten Schicht weniger als 30 %, besonders bevorzugt weniger als 25 % der Gesamtschüttungslänge aller Schichten. Im Allgemeinen liegt die Mindestschüttungslänge dieser Schicht bei 16 %.

Die Schüttungslänge der ersten Katalysatorschicht (Oberschicht) in einem 3-Schichtenkatalysatorsystem macht vorzugsweise 27 bis 60 %, insbesondere 40 bis 55 % der gesamten Katalysatorfüllhöhe im Reaktor aus. Die Schüttungslänge der Mittelschicht macht vorzugsweise 15 bis 55 %, bevorzugt 20 bis 40 % der Gesamtschüttungslänge aus. Bei einem 4-Schichtenkatalysatorsystem macht die Oberschicht vorteilhaft 27 bis 55 %, insbesondere 32 bis 47 %, die obere Mittelschicht 1 vorteilhaft 5 bis 22 %, bevorzugt 8 bis 18 % und die untere Mittelschicht 2 vorteilhaft 8 bis 35 %, insbesondere 12 bis 30 %, der Gesamtschüttungshöhe im Reaktor aus. Die Katalysatorschichten können auch gegebenenfalls auf mehrere Reaktoren verteilt werden. Typische Reaktoren weisen eine Füllhöhe von 2,5 bis 3,4 Metern auf.

Bevorzugt enthält die letzte, unterste Katalysatorschicht mehr als 15 Gew.-% Vanadium (berechnet als V₂O₅) bezogen auf die Aktivmasse des Katalysators, insbesondere 18 bis 22 Gew.-%.

Das Verhältnis von Vanadium (berechnet als V₂O₅) zu Phosphor (berechnet als P) in der letzten Katalysatorschicht ist vorteilhaft etwa 40 und größer als 40, bevorzugt 45 bis 100, besonders bevorzugt 50 bis 70.

Der Alkaligehalt in der Aktivmasse nimmt vorteilhaft von der obersten Katalysatorschicht zu den mittleren Schichten ab. In der letzten Katalysatorschicht ist vorteilhaft kein Alkali enthalten. In der ersten, obersten Katalysatorschicht ist der Alkaligehalt vorteilhaft kleiner als 1,1 Gew.-% Alkali (berechnet als Alkalimetall) bezogen auf die Aktivmasse des Katalysators, bevorzugt liegt der Alkaligehalt im Bereich von 0,1 bis 0,8 Gew.-% Alkali. In der oder den mittleren Katalysatorschichten liegt der Gehalt an Alkali vorteilhaft bei 0,05 bis 0,6 Gew.-% (berechnet als Alkalimetall) bezogen auf die Aktivmasse des Katalysators, insbesondere 0,05 bis 0,3 Gew.-%. Als Alkali wird bevorzugt Cäsium verwendet. Alkali in der Aktivmasse kann teilweise durch Erdalkali wie beispielsweise Barium, Calcium oder Magnesium ersetzt werden.

Die BET-Oberfläche der katalytisch aktiven Komponenten des Katalysators liegt vorteilhaft im Bereich von 5 bis 50 m²/g, bevorzugt 5 bis 30 m²/g, insbesondere 9 bis 27 m²/g.

Der Aktivmassenanteil liegt vorzugsweise bei 3 bis 15 Gew.-%, insbesondere bei 4 bis 12 Gew.-%, bezogen auf die Gesamtkatalysatormasse.

Der Gehalt an ADCA ist bei einer Beladung von mindestens 80 g o-Xylol / Nm³ Luft vorteilhaft kleiner 100 ppm, insbesondere kleiner 75 ppm bei einer zum Stand der Technik vergleichbaren oder verbesserten Ausbeute von ca. 113 % (erhaltenes Phthalsäureanhydrid in Gewichtsprozent bezogen auf 100 %iges o-Xylol). Der Gehalt an Benzoyl-PSA ist vorteilhaft kleiner als 20 ppm, insbesondere kleiner als 15 ppm.

Nach einer bevorzugten Ausführungsform eines Drei-Schichtenkatalysatorsystems weist
a) der am geringsten aktive Katalysator auf nicht porösem und/oder porösem Trägermaterial 7 bis 10 Gew.-%, bezogen auf den gesamten Katalysator, Aktivmasse aufweist, enthaltend 6 bis 11 Gew.-% V₂O₅, 0 bis 3 Gew.-% Sb₂O₃, 0 Gew.-% P, 0,1 bis 1,1 Gew.-% Alkali (ber. als Alkalimetall) und als Rest TiO₂ in Anatasform,
b) der nächst aktivere Katalysator auf nicht porösem und/oder porösem Trägermaterial 7 bis 12 Gew.-%, bezogen auf den gesamten Katalysator, Aktivmasse aufweist, enthaltend 5 bis 13 Gew.-% V₂O₅, 0 bis 3 Gew.-% Sb₂O₃, 0 Gew.-% P, 0 bis 0,4 Gew.-% Alkali (ber. als Alkalimetall) und als Rest TiO₂ in Anatasform,
c) und der aktivste Katalysator auf nicht porösem und/oder porösem Trägermaterial 8 bis 12 Gew.-%, bezogen auf den gesamten Katalysator, Aktivmasse aufweist, enthaltend 10 bis 30 Gew.-% V₂O₅, 0 bis 3 Gew.-% Sb₂O₃, 0 bis 0,43 Gew.-% P, 0 bis 0,1 Gew.-% Alkali (ber. als Alkalimetall) und als Rest TiO₂ in Anatasform,
wobei als Alkalimetall bevorzugt Caesium verwendet wird.

Das eingesetzte Titandioxid in Anatasform weist vorteilhaft eine BET Oberfläche von 5 bis 50 m²/g auf, insbesondere 15 bis 30 m²/g. Es können auch Mischungen von Titandioxid in Anatasform mit unterschiedlicher BET Oberfläche eingesetzt werden, mit der Maßgabe, dass die resultierende BET-Oberfläche einen Wert von 15 bis 30 m²/g aufweist. Die einzelnen Katalysatorschichten können auch Titandioxid mit unterschiedlichen BET-Oberflächen aufweisen. Bevorzugt nimmt die BET-Oberfläche des eingesetzten Titandioxids von der Oberschicht a) zur Unterschicht c) zu.

Nach einer bevorzugten Ausführungsform eines Vier-Schichtenkatalysatorsystems weist
a) der am geringsten aktive Katalysator auf nicht porösem und/oder porösem Trägermaterial 7 bis 10 Gew.-%, bezogen auf den gesamten Katalysator, Aktivmasse aufweist, enthaltend 6 bis 11 Gew.-% V₂O₅, 0 bis 3 Gew.-% Sb₂O₃, 0 Gew.-% P, 0,1 bis 1,1 Gew.-% Alkali (ber. als Alkalimetall) und als Rest TiO₂ in Anatasform,
b1) der nächst aktivere Katalysator auf nicht porösem und/oder porösem Trägermaterial 7 bis 12 Gew.-%, bezogen auf den gesamten Katalysator, Aktivmasse aufweist, enthaltend 4 bis 15 Gew.-% V₂O₅, 0 bis 3 Gew.-% Sb₂O₃, 0 Gew.-% P, 0,1 bis 1 Gew.-% Alkali (ber. als Alkalimetall) und als Rest TiO₂ in Anatasform,
b2) der nächst aktivere Katalysator auf nicht porösem und/oder porösem Trägermaterial 7 bis 12 Gew.-%, bezogen auf den gesamten Katalysator, Aktivmasse aufweist, enthaltend 5 bis 15 Gew.-% V₂O₅, 0 bis 3 Gew.-% Sb₂O₃, 0 Gew.-% P, 0 bis 0,4 Gew.-% Alkali (ber. als Alkalimetall) und als Rest TiO₂ in Anatasform,
c) und der aktivste Katalysator auf nicht porösem und/oder poröse Trägermaterial 8 bis 12 Gew.-%, bezogen auf den gesamten Katalysator, Aktivmasse aufweist, enthaltend 10 bis 30 Gew.-% V₂O₅, 0 bis 3 Gew.-% Sb₂O₃, 0 bis 0,43 Gew.-% P, 0 bis 0,1 Gew.-% Alkali (ber. als Alkalimetall) und als Rest TiO₂ in Anatasform,
wobei als Alkalimetall bevorzugt Caesium verwendet wird.

Bevorzugt werden drei bis fünf Schichten verwendet.

Allgemein können die Katalysatorschichten, z.B. a), b1), b2) und/oder c), auch so angeordnet sein, dass sie jeweils aus zwei oder mehreren Schichten bestehen. Diese Zwischenschichten haben vorteilhaft intermediate Katalysatorzusammensetzungen.

Anstelle von gegeneinander abgegrenzter Schichten der verschiedenen Katalysatoren kann auch ein quasi-kontinuierlicher Übergang der Schichten und ein quasi-gleichmäßiger Anstieg der Aktivität dadurch bewirkt werden, dass man beim Übergang von einer Schicht zur nächsten Schicht eine Zone mit einer Vermischung der aufeinander folgenden Katalysatoren vornimmt.

Als Katalysatoren sind oxidische Trägerkatalysatoren geeignet. Zur Herstellung von Phthalsäureanhydrid durch Gasphasenoxidation von o-Xylol oder Naphthalin oder Gemische davon verwendet man in der Regel kugelförmige, ringförmige oder schalenförmige Träger aus einem Silikat, Siliciumcarbid, Porzellan, Aluminiumoxid, Magnesiumoxid, Zinndioxid, Rutil, Aluminiumsilikat, Magnesiumsilicat (Steatit), Zirkoniumsilicat oder Cersilicat oder Mischungen davon. Besonders bewährt haben sich sogenannte Schalenkatalysatoren, bei denen die katalytisch aktive Masse schalenförmig auf den Träger aufgebracht ist. In der Aktivmasse liegt neben Titandioxid als katalytisch aktiver Bestandteil vorzugsweise Vanadiumpentoxid vor. Weiter können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Derartige Promotoren sind beispielsweise die Alkalimetalloxide, Thallium(I)oxid, Aluminiumoxid, Zirkoniumoxid, Eisenoxid, Nickeloxid, Cobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid. Die Alkalimetalloxide wirken beispielsweise als die Aktivität vermindernde und die Selektivität erhöhende Promotoren. Weiterhin können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei Bindermengen von 3 bis 20 Gew.-%, bezogen auf den Feststoffgehalt der Lösung der Aktivmassenbestandteile, eingesetzt wurden (EP-A 744 214). Bevorzugt werden organische Binder wie in der DE-A 198 24 532 beschrieben verwendet. Wird die katalytisch aktive Masse ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 450°C (DE-A 21 06 796). Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Die Katalysatoren werden zur Reaktion schichtweise in die Rohre eines Rohbündelreaktors gefüllt. Der am geringsten aktive Katalysator wird so im Festbett angeordnet, dass das Reaktionsgas zuerst mit diesem Katalysator und erst im Anschluss mit dem nächst aktiveren Katalysator in der folgenden Schicht in Kontakt kommt. Anschließend kommt das Reaktionsgas mit den noch aktiveren Katalysatorschichten in Kontakt. Die unterschiedlich aktiven Katalysatoren können auf die gleiche oder auf unterschiedliche Temperaturen thermostatisiert werden.

Über die so bereitete Katalysatorschüttung wird das Reaktionsgas bei Temperaturen von im allgemeinen 300 bis 450°C, vorzugsweise 320 bis 420°C und besonders bevorzugt von 340 bis 400°C geleitet. Es wird vorteilhaft ein Überdruck von im allgemeinen 0,1 bis 2,5 bar, vorzugsweise von 0,3 bis 1,5 bar, verwendet. Die Raumgeschwindigkeit liegt im allgemeinen bei 750 bis 5000 h⁻¹.

Das dem Katalysator zugeführte Reaktionsgas (Ausgangsgasgemisch) wird im allgemeinen durch Vermischen von einem molekularen Sauerstoff enthaltenden Gas, das außer Sauerstoff noch geeignete Reaktionsmoderatoren, wie Stickstoff und/oder Verdünnungsmittel, wie Dampf und/oder Kohlendioxid, enthalten kann, mit dem zu oxidierenden, aromatischen Kohlenwasserstoff erzeugt. Das molekularen Sauerstoff enthaltende Gas kann im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten. Zur Erzeugung des Reaktionsgases wird das molekularen Sauerstoff enthaltende Gas im allgemeinen mit 30 g bis 150 g je Nm³ Gas, insbesondere mit 60 bis 120 g je Nm³, des zu oxidierenden, aromatischen Kohlenwasserstoffs beschickt.

Im Allgemeinen erfolgt die Umsetzung so, dass in der ersten Reaktionszone der größte Teil des im Reaktionsgas enthaltenen o-Xylols und/oder Naphthalins umgesetzt wird.

Die Hot-Spot-Temperatur der obersten Schicht beträgt vorzugsweise 400 bis 470°C, in der oder den Mittelschichten eines mehrlagigen Katalysatorsystems ist sie vorteilhaft kleiner als 420°C, insbesondere kleiner 410°C.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 beschrieben ist. Als Katalysator verwendet man dabei vorzugsweise einen noch aktiveren Katalysator als den der untersten Schicht.

Phthalsäureanhydrid lässt sich erfindungsgemäß auch bei hohen Beladungen mit o-Xylol und/oder Naphthalin in hoher Ausbeute mit einer geringen Konzentration an Nebenprodukten, insbesondere einer sehr geringen Menge an ADCA und Benzoyl-PSA, herstellen.

### Beispiele:

### A. Herstellung der Katalysatoren

### A.1 Herstellung des Katalysators 1 (3-Schichtenkatalysator)

### Oberschicht (a)

35,32 g Anatas (TiO₂-1, BET-OF 9 m²/g), 65,58 g Anatas (TiO₂-2, BET-OF 20 m²/g), 7,97 g Vanadiumpentoxid, 2,65 g Antimonoxid, 0,45 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, Außendurchmesser (AD) x Länge (L) x Innendurchmesser (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,33 Gew.-% Cäsium (berechnet als Cs), 31,54 Gew.-% Titandioxid (TiO₂-1) und 58,56 Gew.-% Titandioxid (TiO₂-2).

### Mittelschicht (b)

24,16 g Anatas (TiO₂-1, BET-OF 9 m²/g), 72,48 g Anatas (TiO₂-2, BET-OF 20 m²/g), 7,74 g Vanadiumpentoxid, 2,57 g Antimontrioxid, 0,13 g Cäsiumcarbonat wurden in 650 ml entionisiertem Wasser suspendiert und 18 Stunden gerührt, um eine homogene Verteilung zu erzielen. Dieser Suspension wurden 60 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,10 Gew.-% Cäsium (berechnet als Cs), 22,23 Gew.-% Titandioxid (TiO₂-1) und 66,68 Gew.-% Titandioxid (TiO₂-2).

### Unterschicht (c)

22,07 g Anatas (TiO₂-1, BET-OF 9 m²/g), 88,28 g Anatas (TiO₂-2, BET-OF 27 m²/g), 28,07 g Vanadiumpentoxid, 1,93 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 20,0 Gew.-% Vanadium (berechnet als V₂O₅), 0,37 Gew.-% Phosphor (berechnet als P), 15,73 Gew.-% Titandioxid (TiO₂-1) und 62,90 Gew.-% Titandioxid (TiO₂-2).

### A.2. Herstellung des Katalysators 2 (4-Schichtenkatalysator)

### Oberschicht (a)

29,27 g Anatas (TiO₂-1, BET-OF 9 m²/g), 69,80 g Anatas (TiO₂-2, BET-OF 21 m²/g), 7,83 g Vanadiumpentoxid, 2,61 g Antimonoxid, 0,49 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,36 Gew.-% Cäsium (berechnet als Cs), 27,20 Gew.-% Titandioxid (TiO₂-1) und 63,46 Gew.-% Titandioxid (TiO₂-2).

### Mittelschicht 1 (b1)

24,61 g Anatas (TiO₂-1, BET-OF 9 m²/g), 74,46 g Anatas (TiO₂-2, BET-OF 21 m²/g), 7,82 g Vanadiumpentoxid, 2,60 g Antimonoxid, 0,35 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,26 Gew.-% Cäsium (berechnet als Cs), 22,60 Gew.-% Titandioxid (TiO₂-1) und 67,79 Gew.-% Titandioxid (TiO₂-2).

### Mittelschicht 2 (b2)

24,82 g Anatas (TiO₂-1, BET-OF 9 m²/g), 74,46 g Anatas (TiO₂-2, BET-OF 21 m²/g), 7,82 g Vanadiumpentoxid, 2,60 g Antimonoxid, 0,135 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,10 Gew.-% Cäsium (berechnet als Cs), 22,60 Gew.-% Titandioxid (TiO₂-1) und 67,79 Gew.-% Titandioxid (TiO₂-2).

### Unterschicht (c)

17,23 g Anatas (TiO₂-1, BET-OF 9 m²/g), 69,09 g Anatas (TiO₂-2, BET-OF 27 m²/g), 21,97 g Vanadiumpentoxid, 1,55 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 20,0 Gew.-% Vanadium (berechnet als V₂O₅), 0,38 Gew.-% Phosphor (berechnet als P), 15,73 Gew.-% Titandioxid (TiO₂-1) und 62,90 Gew.-% Titandioxid (TiO₂-3).

### A.3. Herstellung des Katalysators 3 (P-dotierte Mittelschicht - 3-Schichtenkatalysator - Vergleichsbeispiel 1)

### Oberschicht (a)

35,32 g Anatas (TiO₂-1, BET-OF 9 m²/g), 65,58 g Anatas (TiO₂-2, BET-OF 20 m²/g), 7,97 g Vanadiumpentoxid, 2,65 g Antimonoxid, 0,45 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 Gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,33 Gew.-% Cäsium (berechnet als Cs), 31,54 Gew.-% Titandioxid (TiO₂-1) und 58,56 Gew.-% Titandioxid (TiO₂-2).

### Mittelschicht (b)

34,32 g Anatas (TiO₂-1, BET-OF 9 m²/g), 102,90 g Anatas (TiO₂-2, BET-OF 20 m²/g), 10,99 g Vanadiumpentoxid, 3,66 g Antimontrioxid, 2,30 g Ammoniumdihydrogenphosphat und 0,19 g Cäsiumcarbonat wurden in 650 ml entionisiertem Wasser suspendiert und 18 Stunden gerührt, um eine homogene Verteilung zu erzielen. Dieser Suspension wurden 52 g organische Binder, bestehend aus einem Copolymer aus Vinylacetat und Vinyllaurat in Form einer 50 Gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,40 Gew.-% Phosphor (berechnet als P), 0,10 Gew.-% Cäsium (berechnet als Cs), 22,23 Gew.-% Titandioxid (TiO₂-1) und 66,68 Gew.-% Titandioxid (TiO₂-2).

### Unterschicht (c)

22,07 g Anatas (TiO₂-1, BET-OF 9 m²/g), 88,28 g Anatas (TiO₂-3, BET-OF 27 m²/g), 28,07 g Vanadiumpentoxid, 1,93 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 20,0 Gew.-% Vanadium (berechnet als V₂O₅), 0,37 Gew.-% Phosphor (berechnet als P), 15,73 Gew.-% Titandioxid (TiO₂-1) und 62,90 Gew.-% Titandioxid (TiO₂-3).

### A.4. Herstellung des Katalysators 4 (P-dotierte Oberschicht und Mittelschicht - 3-Schichtenkatalysator - Vergleichsbeispiel 2)

### Oberschicht (a)

35,32 g Anatas (TiO₂-1, BET-OF 9 m²/g), 67,96 g Anatas (TiO₂-2, BET-OF 20 m²/g), 8,26 g Vanadiumpentoxid, 2,75 g Antimonoxid, 1,29 g Ammoniumdihydrogenphosphat, 0,47 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,30 Gew.-% Phosphor (berechnet als P), 0,33 Gew.-% Cäsium (berechnet als Cs), 31,54 Gew.-% Titandioxid (TiO₂-1) und 58,56 Gew.-% Titandioxid (TiO₂-2).

### Mittelschicht (b)

34,32 g Anatas (TiO₂-1, BET-OF 9 m²/g), 102,90 g Anatas (TiO₂-2, BET-OF 20 m²/g), 10,99 g Vanadiumpentoxid, 3,66 g Antimontrioxid, 2,30 g Ammoniumdihydrogenphosphat und 0,19 g Cäsiumcarbonat wurden in 650 ml entionisiertem Wasser suspendiert und 18 Stunden gerührt, um eine homogene Verteilung zu erzielen. Dieser Suspension wurden 52 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,40 Gew.-% Phosphor (berechnet als P), 0,10 Gew.-% Cäsium (berechnet als Cs), 22,23 Gew.-% Titandioxid (TiO₂-1) und 66,68 Gew.-% Titandioxid (TiO₂-2).

### Unterschicht (c)

22,07 g Anatas (TiO₂-1, BET-OF 9 m²/g), 88,28 g Anatas (TiO₂-3, BET-OF 27 m²/g), 28,07 g Vanadiumpentoxid, 1,93 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10% des Gesamtgewichtes des fertigen Katalysators. Die auf diese Weise aufgebrachte katalytisch aktive Masse, also die Katalysatorschale, enthielt nach einer Stunde Calcination auf 450°C 20,0 Gew.-% Vanadium (berechnet als V₂O₅), 0,37 Gew.-% Phosphor (berechnet als P), 15,73 Gew.-% Titandioxid (TiO₂-1) und 62,90 Gew.-% Titandioxid (TiO₂-3).

### A.5. Herstellung des Katalysators 5 (7,12 Gew.-% V₂O₅ in Schicht (c), V₂O₅ / P = 19,2 - 3-Schichtenkatalysator - Vergleichsbeispiel 3)

### Oberschicht (a)

35,32 g Anatas (TiO₂-1, BET-OF 9 m²/g), 65,58 g Anatas (TiO₂-2, BET-OF 20 m²/g), 7,97 g Vanadiumpentoxid, 2,65 g Antimonoxid, 0,45 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,33 Gew.-% Cäsium (berechnet als Cs), 31,54 Gew.-% Titandioxid (TiO₂-1) und 58,56 Gew.-% Titandioxid (TiO₂-2).

### Mittelschicht (b)

24,16 g Anatas (TiO₂-1, BET-OF 9 m²/g), 72,48 g Anatas (TiO₂-2, BET-OF 20 m²/g), 7,74 g Vanadiumpentoxid, 2,57 g Antimontrioxid, 0,13 g Cäsiumcarbonat wurden in 650 ml entionisiertem Wasser suspendiert und 18 Stunden gerührt, um eine homogene Verteilung zu erzielen. Dieser Suspension wurden 60 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,10 Gew.-% Cäsium (berechnet als Cs), 22,23 Gew.-% Titandioxid (TiO₂-1) und 66,68 Gew.-% Titandioxid (TiO₂-2).

### Unterschicht (c)

40,15 g Anatas (TiO₂-1, BET-OF 9 m²/g), 88,28 g Anatas (TiO₂-3, BET-OF 27 m²/g), 9,99 g Vanadiumpentoxid, 1,93 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 0,37 Gew.-% Phosphor (berechnet als P), 15,73 Gew.-% Titandioxid (TiO₂-1) und 62,90 Gew.-% Titandioxid (TiO₂-3).

### A.6 Herstellung des Katalysators 6 (11,5 Gew.-% V₂O₅ in Schicht (c), V₂O₅ / P = 31,1 - 3-Schichtenkatalysator - Vergleichsbeispiel 4)

### Oberschicht (a)

35,32 g Anatas (TiO₂-1, BET-OF 9 m²/g), 65,58 g Anatas (TiO₂-2, BET-OF 20 m²/g), 7,97 g Vanadiumpentoxid, 2,65 g Antimonoxid, 0,45 g Cäsiumcarbonat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 8 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,33 Gew.-% Cäsium (berechnet als Cs), 31,54 Gew.-% Titandioxid (TiO₂-1) und 58,56 Gew.-% Titandioxid (TiO₂-2).

### Mittelschicht (b)

24,16 g Anatas (TiO₂-1, BET-OF 9 m²/g), 72,48 g Anatas (TiO₂-2, BET-OF 20 m²/g), 7,74 g Vanadiumpentoxid, 2,57 g Antimontrioxid, 0,13 g Cäsiumcarbonat wurden in 650 ml entionisiertem Wasser suspendiert und 18 Stunden gerührt, um eine homogene Verteilung zu erzielen. Dieser Suspension wurden 60 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 7,12 Gew.-% Vanadium (berechnet als V₂O₅), 2,37 Gew.-% Antimon (berechnet als Sb₂O₃), 0,10 Gew.-% Cäsium (berechnet als Cs), 22,23 Gew.-% Titandioxid (TiO₂-1) und 66,68 Gew.% Titandioxid (TiO₂-2).

### Unterschicht (c)

34,00 g Anatas (TiO₂-1, BET-OF 9 m²/g), 88,28 g Anatas (TiO₂-3, BET-OF 27 m²/g), 16,14 g Vanadiumpentoxid, 1,93 g Ammoniumdihydrogenphosphat wurden in 650 ml entionisiertem Wasser suspendiert und 18 Stunden gerührt, um eine homogene Verteilung zu erzielen. Dieser Suspension wurden 50 g organische Binder, bestehend aus einem Copolymer aus Vinylacetat und Vinyllaurat in Form einer 50 gew.-%igen wässrigen Dispersion, zugegeben. Die erhaltene Suspension wurde anschließend auf 1200 g Steatit (Magnesiumsilikat) in Form von Ringen (7 x 7 x 4 mm, (AD) x (L) x (ID)) aufgesprüht und getrocknet. Das Gewicht der aufgetragenen Schale betrug 10 % des Gesamtgewichtes des fertigen Katalysators. Die auf diese Weise aufgebrachte katalytisch aktive Masse, also die Katalysatorschale, enthielt nach einer Stunde Calcination auf 450°C 11,50 Gew.-% Vanadium (berechnet als V₂O₅), 0,37 Gew.-% Phosphor (berechnet als P), 15,73 Gew.-% Titandioxid (TiO₂-1) und 62,90 Gew.-% Titandioxid (TiO₂-3).

### B Oxidation von o-Xylol zu PSA

### B.1 3-Schichtenkatalysator

Von unten nach oben wurden jeweils 0,70 m des Katalysators der Unterschicht (c), 0,60 m des Katalysators der Mittelschicht (b) und 1,50 m des Katalysators der Oberschicht (a) in ein 3,85 m langes Eisenrohr mit einer lichten Weite von 25 mm eingefüllt. Das Eisenrohr war zur Temperaturregelung von einer Salzschmelze umgeben, eine 2 mm Thermohülse mit eingebautem Zugelement diente der Katalysatortemperaturmessung. Durch das Rohr wurden stündlich von oben nach unten 4 Nm³-Luft mit Beladungen an 98,5 gew.-%igem o-Xylol von 0 bis 100 g/Nm³ geleitet. Nach einer Laufzeit von 10-14 Tagen wird die o-Xylol-Zufuhr unterbrochen und der Katalysator über einen Zeitraum von 72 Stunden folgenden Bedingungen ausgesetzt: Salzbadtemperatur (SBT) von 410°C, Luft 2 Nm³/h. Dabei wurden bei 60-100 g o-Xylol/Nm³ die in Tabelle 1 und 2 zusammengefaßten Ergebnisse erhalten ("PSA-Aubeute" bedeutet das erhaltene PSA in Gewichtprozent, bezogen auf 100%iges o-Xylol).

### B.2 4-Schichtenkatalysator

Von unten nach oben wurden jeweils 0,70 m des Katalysators der Unterschicht (c), 0,70 m des Katalysators der Mittelschicht 2 (b2), 0,50 m des Katalysators der Mittelschicht 1 (b1) und 1,30 m des Katalysators der Oberschicht (a) in ein 3,85 m langes

Eisenrohr mit einer lichten Weite von 25 mm eingefüllt. Im übrigen erfolgte die Versuchsdurchführung wie in B.1 angegeben.

Die Versuchsergebnisse nach der Aktivierung sind in Tabelle 1 und 2 zusammengestellt.

Folgende Abkürzungen wurden verwendet:

| | |
|---|---|
| HST | Hot-Spot-Temperatur |
| OS | Oberschicht |
| MS | Mittelschicht |
| US | Unterschicht |
| ROG | Reaktion-Outlet-Gas |
| SBT | Salzbadtemperatur |
| PHD | Phthalid |
| PSA | Phthalsäureanhydrid |
| Benzoyl-PSA | 4-Benzoylphthalsäureanhydrid |
| ADCA | Anthrachinondicarbonsäureanhydrid |

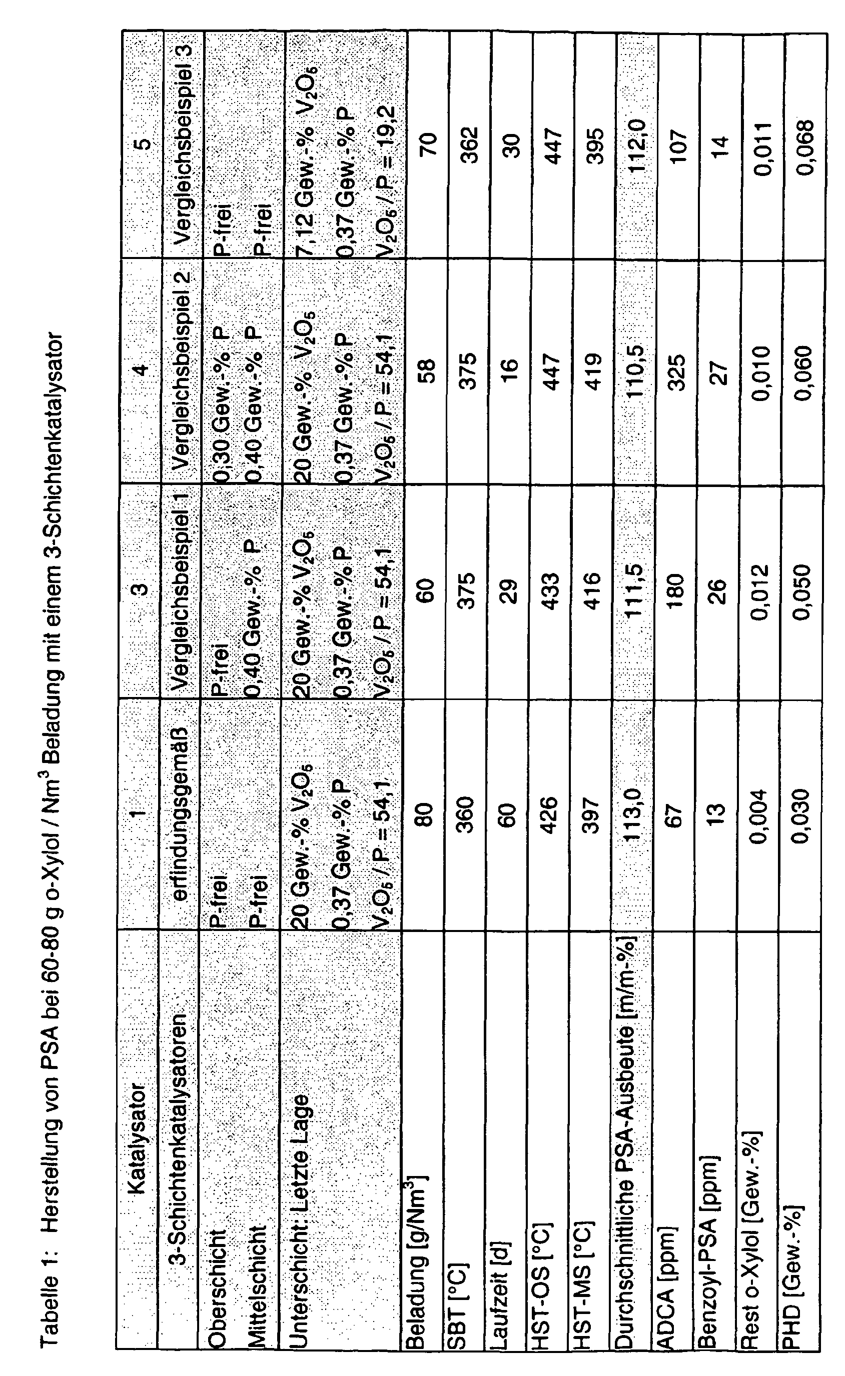

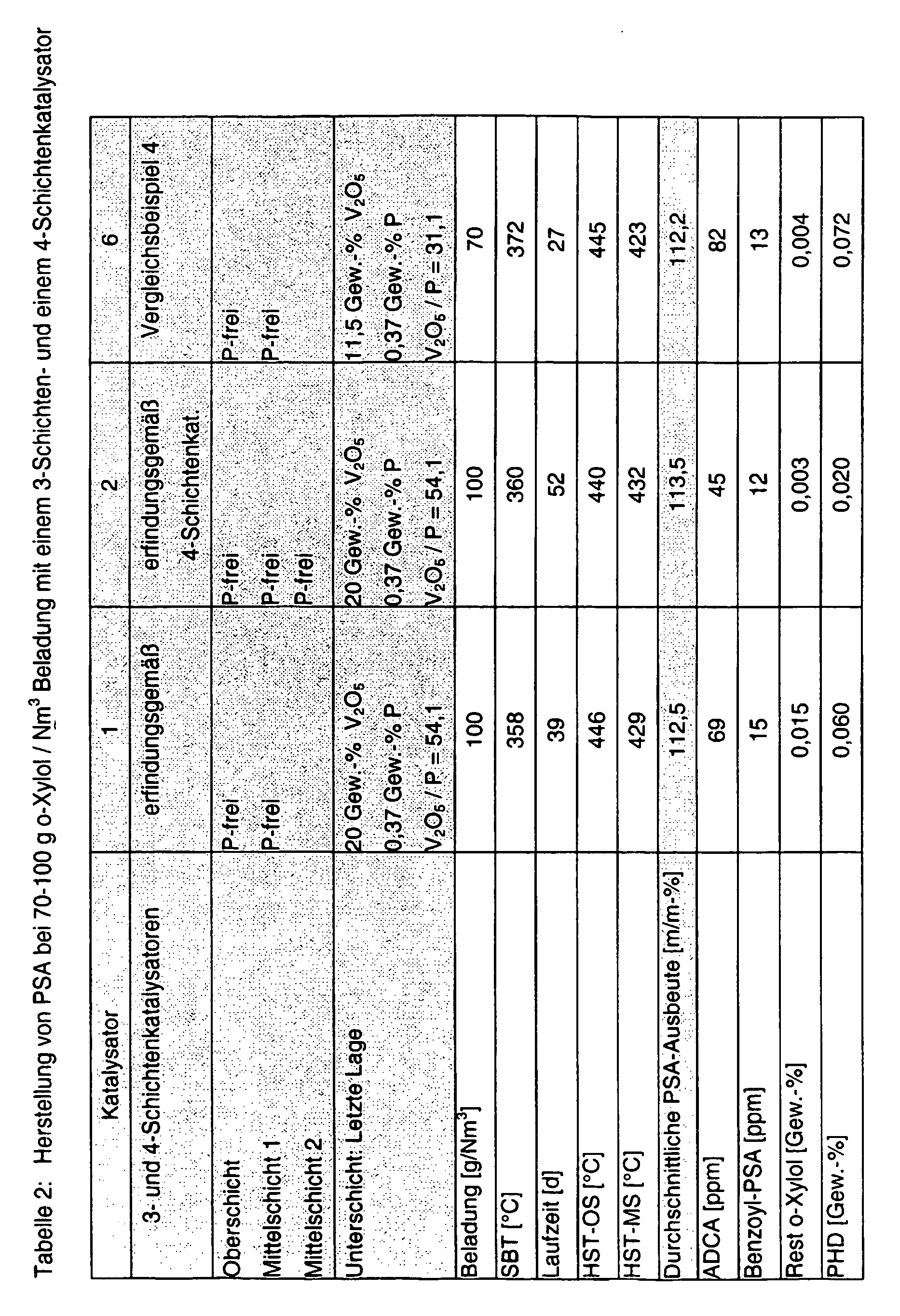

Zusammensetzung der Katalysatoren (%-Angaben = Gew.-%)

| Katalysator 1 | | | |
|---|---|---|---|
| Katalysator | Oberschicht | Mittelschicht | Unterschicht |
| Aktivmasse [%] | 8 | 10 | 10 |
| TiO₂-1, 9 m²/g [%] | 31,54 | 22,23 | 15,73 |
| TiO₂-2, 20 m²/g [%] | 58,56 | 66,68 | - |
| TiO₂-3, 27m²/g [%] | - | - | 62,90 |
| V₂O₅ [%] | 7,12 | 7,12 | 20,0 |
| Sb₂O₃ [%] | 2,37 | 2,37 | - |
| Cs [%] | 0,33 | 0,10 | - |
| P [%] | - | - | 0,37 |

| Katalysator 2 | | | | |
|---|---|---|---|---|
| Katalysator | Oberschicht | Mittelschicht 1 | Mittelschicht 2 | Mittelschicht 3 |
| Aktivmasse [%] | 8 | 8 | 8 | 8 |
| TiO₂-1, 9 m²/g [%] | 27,20 | 22,60 | 22,60 | 15,73 |
| TiO₂-2, 20 m²/g [%] | 63,46 | 67,79 | 67,79 | - |
| TiO₂-3, 27m²/g [%] | - | - | - | 62,90 |
| V₂O₅ [%] | 7,12 | 7,12 | 7,12 | 20,0 |
| 2,37Sb₂O₃ [%] | 2,37 | 2,37 | - | |
| Cs [%] | 0,33 | 0,25 | 0,10 | - |
| P [%] | - | - | - | 0,37 |

| Katalysator 3 - Vergleichsbeispiel | | | |
|---|---|---|---|
| Katalysator | Oberschicht | Mittelschicht | Unterschicht |
| Aktivmasse [%] | 8 | 10 | 10 |
| TiO₂-1, 9 m²/g [%] | 31,54 | 22,23 | 15,73 |
| TiO₂-2, 20 m²/g [%] | 58,56 | 66,68 | - |
| TiO₂-3, 27m²/g [%] | - | - | 62,90 |
| V₂O₅ [%] | 7,12 | 7,12 | 20,0 |
| Sb₂O₃ [%] | 2,37 | 2,37 | - |
| Cs [%] | 0,33 | 0,10 | - |
| P [%] | - | 0,40 | 0,37 |

| Katalysator 4- Vergleichsbeispiel | | | |
|---|---|---|---|
| Katalysator | Oberschicht | Mittelschicht | Unterschicht |
| Aktivmasse [%] | 8 | 10 | 10 |
| TiO₂-1, 9 m²/g [%] | 31,54 | 22,23 | 15,73 |
| TiO₂-2, 20 m²/g [%] | 58,56 | 66,68 | - |
| TiO₂-3, 27m²/g [%] | - | - | 62,9 |
| V₂O₅ [%] | 7,12 | 7,12 | 20,0 |
| Sb₂O₃ [%] | 2,37 | 2,37 | - |
| Cs [%] | 0,33 | 0,10 | - |
| P [%] | 0,30 | 0,40 | 0,37 |

| Katalysator 5- Vergleichsbeispiel | | | |
|---|---|---|---|
| Katalysator | Oberschicht | Mittelschicht | Unterschicht |
| Aktivmasse [%] | 8 | 10 | 10 |
| TiO₂-1, 9 m²/g [%] | 31,54 | 22,23 | 15,73 |
| TiO₂-2, 20 m²/g [%] | 58,56 | 66,68 | - |
| TiO₂-3, 27m²/g [%] | - | - | 62,90 |
| V₂O₅ [%] | 7,12 | 7,12 | 7,12 |
| Sb₂O₃ [%] | 2,37 | 2,37 | - |
| Cs [%] | 0,33 | 0,10 | - |
| P [%] | - | - | 0,37 |

| Katalysator 6 - Vergleichsbeispiel | | | |
|---|---|---|---|
| Katalysator | Oberschicht | Mittelschicht | Unterschicht |
| Aktivmasse [%] | 8 | 10 | 10 |
| TiO₂-1, 9 m²/g [%] | 31,54 | 22,23 | 15,73 |
| TiO₂-2, 20 m²/g [%] | 58,56 | 66,68 | - |
| TiO₂-3, 27m²/g [%] | - | - | 62,90 |
| V₂O₅ [%] | 7,12 | 7,12 | 11,50 |
| Sb₂O₃ [%] | 2,37 | 2,37 | - |
| Cs [%] | 0,33 | 0,10 | - |
| P [%] | - | - | 0,37 |

## Patentansprüche

1. Verfahren zur Herstellung von Phthalsäureanhydrid durch katalytische Gasphasenoxidation von Xylol und/oder Naphthalin mit einem molekularen Sauerstoff enthaltenden Gas in einem Festbett und mittels mindestens drei in Schichten übereinander angeordneter Katalysatoren, deren Aktivität von Schicht zu Schicht von der Gaseintrittsseite zur Gasaustrittsseite ansteigt, auf deren Kern aus Trägermaterial eine Schicht aus katalytisch aktiven Metalloxiden aufgebracht ist, **dadurch gekennzeichnet, dass** nur die letzte Katalysatorschicht Phosphor aufweist, dass in der letzten Schicht mindestens 10 Gew.-% Vanadium (berechnet als V₂O₅) bezogen auf die Aktivmasse des Katalysators vorliegen und dass das Verhältnis Vanadium (berechnet als V₂O₅) zu Phosphor einen Wert von größer als 35 aufweist.

2. Verfahren nach Anspruch 1, **dadurch gekennzeichnet, dass** die Schüttungslänge der letzten Katalysatorschicht höchstens 40 % der Gesamtschüttungslänge aller Schichten beträgt.

3. Verfahren nach Anspruch 1, **dadurch gekennzeichnet, dass** die Schüttungslänge der letzten Katalysatorschicht höchstens 25 % der Gesamtschüttungslänge aller Schichten beträgt.

4. Verfahren nach den Ansprüchen 1 bis 3, **dadurch gekennzeichnet, dass** das Verhältnis Vanadium (berechnet als V₂O₅) zu Phosphor in der letzten Katalysatorschicht einen Wert von 40 bis 100 aufweist.

5. Verfahren nach den Ansprüchen 1 bis 4, **dadurch gekennzeichnet, dass** die letzte Katalysatorschicht mindestens 15 Gew.-% Vanadium (berechnet als V₂O₅) bezogen auf die Aktivmasse des Katalysators aufweist.

6. Verfahren nach den Ansprüchen 1 bis 5, **dadurch gekennzeichnet, dass** die letzte Katalysatorschicht 18 bis 22 Gew.-% Vanadium (berechnet als V₂O₅) bezogen auf die Aktivmasse des Katalysators aufweist.

7. Verfahren nach den Ansprüchen 1 bis 6, **dadurch gekennzeichnet, dass** die erste Katalysatorschicht weniger als 1,1 Gew.-% Alkali (berechnet als Alkalimetall) bezogen auf die Aktivmasse des Katalysators aufweist.

8. Verfahren nach den Ansprüche 1 bis 7, **dadurch gekennzeichnet, dass** die erste Katalysatorschicht einen Alkaligehalt von 0,1 bis 0,8 Gew.-% Alkali (berechnet als Alkalimetall) bezogen auf die Aktivmasse und die mittlere(n) Katalysatorschicht(en) einen Alkaligehalt von 0,05 bis 0,6 Gew.-% (berechnet als Alkalimetall) aufweisen.

9. Verfahren nach den Ansprüchen 1 bis 8, **dadurch gekennzeichnet, dass** man Cäsium als Alkalimetallzusatz verwendet.

10. Verfahren nach den Ansprüchen 1 bis 9, **dadurch gekennzeichnet, dass** die letzte Katalysatorschicht weniger als 1 Gew.-% Phosphor aufweist.

11. Verfahren nach den Ansprüchen 1 bis 10, **dadurch gekennzeichnet, dass** die BET-Oberfläche der Aktivmasse einen Wert von 5 bis 50 m²/g aufweist.

12. Verfahren nach den Ansprüchen 1 bis 11, **dadurch gekennzeichnet, dass** der Aktivmassenanteil bezogen auf die Gesamtmasse des Katalysators bei 3 bis 15 Gew.-% liegt.

13. Verfahren nach den Ansprüchen 1 bis 12, **dadurch gekennzeichnet, dass** der Gehalt an Anthrachinondicarbonsäure im Produkt Phthalsäureanhydrid unter 75 ppm liegt.

## Claims

1. A process for preparing phthalic anhydride by catalytic gas-phase oxidation of xylene and/or naphthalene by means of a gas comprising molecular oxygen in a fixed bed using at least three catalysts which are arranged above one another in zones and whose activity increases from zone to zone from the gas inlet end to the gas outlet end and to whose core of support material a layer of catalytically active metal oxides has been applied, wherein only the last catalyst zone comprises phosphorus, at least 10% by weight of vanadium (calculated as V₂O₅) based on the active composition of the catalyst is present in the last zone and the ratio of vanadium (calculated as V₂O₅) to phosphorus is greater than 35.

2. The process according to claim 1, wherein the bed length of the last catalyst zone is not more than 40% of the total bed length of all zones.

3. The process according to claim 1, wherein the bed length of the last catalyst zone is not more than 25% of the total bed length of all zones.

4. The process according to any of claims 1 to 3, wherein the ratio of vanadium (calculated as V₂O₅) to phosphorus in the last catalyst zone is from 40 to 100.

5. The process according to any of claims 1 to 4, wherein the last catalyst zone comprises at least 15% by weight of vanadium (calculated as V₂O₅) based on the active composition of the catalyst.

6. The process according to any of claims 1 to 5, wherein the last catalyst zone comprises from 18 to 22% by weight of vanadium (calculated as V₂O₅) based on the active composition of the catalyst.

7. The process according to any of claims 1 to 6, wherein the first catalyst zone comprises less than 1.1% by weight of alkali (calculated as alkali metal) based on the active composition of the catalyst.

8. The process according to any of claims 1 to 7, wherein the first catalyst zone has an alkali content of from 0.1 to 0.8% by weight of alkali (calculated as alkali metal) based on the active composition and the middle catalyst zone(s) has/have an alkali content of from 0.05 to 0.6% by weight (calculated as alkali metal).

9. The process according to any of claims 1 to 8, wherein cesium is used as alkali metal additive.

10. The process according to any of claims 1 to 9, wherein the last catalyst zone comprises less than 1% by weight of phosphorus.

11. The process according to any of claims 1 to 10, wherein the BET surface area of the active composition is from 5 to 50 m²/g.

12. The process according to any of claims 1 to 11, wherein the proportion of active composition based on the total mass of the catalyst is from 3 to 15% by weight.

13. The process according to any of claims 1 to 12, wherein the anthraquinonedicarboxylic acid content of the phthalic anhydride product is less than 75 ppm.

## Revendications

1. Procédé de préparation d'anhydride phtalique par oxydation en phase gazeuse de xylène et/ou de naphtalène par un gaz contenant de l'oxygène moléculaire, dans un lit fixe et au moyen d'au moins trois catalyseurs qui sont agencés l'un au-dessus de l'autre en couches et dont l'activité croît de couche en couche depuis le côté entrée de gaz jusqu'au côté sortie de gaz, une couche d'oxydes métalliques catalytiquement actifs étant appliquée sur leur noyau à base de matière de support, **caractérisé en ce que** seule la dernière couche de catalyseur présente du phosphore, **en ce que** dans la dernière couche il y a au moins 10 % en poids de vanadium (calculé sous la forme de V₂O₅) par rapport à la masse active du catalyseur et **en ce que** le rapport entre vanadium (calculé sous la forme de V₂O₅) et phosphore présente une valeur supérieure à 35.

2. Procédé suivant la revendication 1, **caractérisé en ce que** la longueur de garnissage de la dernière couche de catalyseur est au maximum de 40 % de la longueur de garnissage totale de toutes les couches.

3. Procédé suivant la revendication 1, **caractérisé en ce que** la longueur de garnissage de la dernière couche de catalyseur est au maximum de 25 % de la longueur de garnissage totale de toutes les couches.

4. Procédé suivant les revendications 1 à 3,
**caractérisé en ce que** le rapport entre vanadium (calculé sous la forme de V₂O₅) et phosphore dans la dernière couche de catalyseur présente une valeur de 40 à 100.

5. Procédé suivant les revendications 1 à 4,
**caractérisé en ce que** la dernière couche de catalyseur présente au moins 15 % en poids de vanadium (calculé sous la forme de V₂O₅) par rapport à la masse active du catalyseur.

6. Procédé suivant les revendications 1 à 5,
**caractérisé en ce que** la dernière couche de catalyseur présente 18 à 22 % en poids de vanadium (calculé sous la forme de V₂O₅) par rapport à la masse active du catalyseur.

7. Procédé suivant les revendications 1 à 6,
**caractérisé en ce que** la première couche de catalyseur présente moins de 1,1 % en poids d'alcali (calculé sous la forme de métal alcalin) par rapport à la masse active du catalyseur.

8. Procédé suivant les revendications 1 à 7,
**caractérisé en ce que** la première couche de catalyseur présente une teneur en alcali de 0,1 à 0,8 % en poids d'alcali (calculé sous la forme de métal alcalin) par rapport à la masse active et la ou les couches de catalyseur médianes une teneur en alcali de 0,05 à 0,6 % en poids (calculé sous la forme de métal alcalin).

9. Procédé suivant les revendications 1 à 8,
**caractérisé en ce qu'**on utilise du césium comme addition de métal alcalin.

10. Procédé suivant les revendications 1 à 9,
**caractérisé en ce que** la dernière couche de catalyseur présente moins de 1 % en poids de phosphore.

11. Procédé suivant les revendications 1 à 10,
**caractérisé en ce que** la surface BET de la masse active présente une valeur de 5 à 50 m²/g.

12. Procédé suivant les revendications 1 à 11,
**caractérisé en ce que** la fraction de masse active par rapport à la masse totale du catalyseur est de 3 à 15 % en poids.

13. Procédé suivant les revendications 1 à 12,
**caractérisé en ce que** la teneur en acide anthraquinonedicarboxylique dans le produit, l'anhydride phtalique, est inférieure à 75 ppm.
